Europäisches Patentamt

European Patent Office

Office européen des brevets

(11) Publication number: **0 362 462**
**A2**

## (12) EUROPEAN PATENT APPLICATION

(21) Application number: 89104839.9

(51) Int. Cl.5: **A61M 25/06**

(22) Date of filing: 17.03.89

(30) Priority: 04.10.88 US 253204
16.12.88 US 285899

(43) Date of publication of application:
**11.04.90 Bulletin 90/15**

(84) Designated Contracting States:
**DE ES FR GB IT SE**

(71) Applicant: **Catheter Technology Corporation**
**3385 West 1820 South**
**Salt Lake City Utah 84104(US)**

(72) Inventor: **Moorehead, Harvey Robert**
**1694 East 5685 South**
**Salt Lake City Utah 84121(US)**
Inventor: **Wiita, Thomas A.**
**467 South 1200 East**
**Salt Lake City Utah 84102(US)**

(74) Representative: **Meddle, Alan Leonard et al**
**FORRESTER & BOEHMERT**
**Widenmayerstrasse 4/I**
**D-8000 München 22(DE)**

(54) **Medical peel away sheath and method of making the same.**

(57) A co-material peel away hollow sheath (10) for temporarily creating a passageway into a desired body site of a patient for placement of one end of an indwelling device (16) at the body site through the sheath, the sheath wall (28) comprising two opposed axially-directed wall segments (36, 38) formed of material which has a relatively low resistance to shear, the remainder (40, 42) of the wall comprising material having a relatively high resistance to shear such that the sheath manually tears axially along the low shear resistant segments into two pieces for removal from the indwelling device.

Fig. I

## Medical peel away sheath and method of making the same

THIS INVENTION relates to a medical peel away sheath, and more particularly to a novel integral bi-material peel away sheath intended to temporarily preserve a tubular passageway from the exterior to a desired body site or internally between two body sites. The invention also relates to a method of making such a peel away sheath.

For many medical purposes, it is important to place devices, including soft, yielding devices, through the skin and underlying tissue layers and into blood vessels or other compartments or locations inside the body of a patient. These include but are not limited to catheters, pacemaker leads and hydrocephalic shunts. Such devices often have one end in the desired location or compartment and the other outside the body, i.e. they are transcutaneous devices because they cross the skin during placement and/or in use. Identical problems occur in installing devices between two compartment within the body, e.g. in placing a catheter between the subcutaneous tissue area and a blood vessel when the catheter is to be used in conjunction with a subcutaneous vascular access portal. Transcutaneous devices are included when the term intercompartmental is used herein. Several methods have been used to place such devices, each with advantages and problems.

Perhaps the earliest and most straightforward approach is to surgically cut an opening through from one compartment into the other compartment, insert the device, then sew up the surgical wound which will self-repair and heal around the device. One example of this is known as the "cut-down" placement of a catheter. In this procedure, the physician cuts through the skin with a scalpel down to a blood vessel, nicks the vessel, inserts the catheter tip through the nick and into the vessel, advances the catheter tip to the desired location, sews the vessel wall tightly around the catheter, and finally sews up the skin incision. One advantage of this method is that generally any device can be placed practically anywhere in the body. Disadvantages include the need for a skilled, highly trained physician as well a specialized medical facilities to perform the procedure safely, and the trauma, disruption and infection risk to the many tissues involved.

To address these disadvantages, percutaneous methods have been developed to create small openings and place such devices through them with minimal tissue disruption, using specialized placement assist devices to reduce the need for skilled personnel and elaborate operating room facilities. One such device is a solid dilator. The key features include a tapered end which will spread aside the tissues at a puncture site as the dilator is advanced through tissue from one compartment to another compartment and a hollow interior passage through which the device can be advanced once the dilator has created the opening. The dilator is then removed over the end of the device. A succession of gradually larger-sized dilators are sometimes used to create a large hole through which a relatively large device can be inserted. If the device is sufficiently firm, stiff and unyielding, the dilator may be used to pre-spread the tissue and create the opening, then withdrawn completely and the stiff device quickly inserted through the opening thus created before it has a chance to reclose.

However, many medical devices, including pacemaker leads, catheters and hydrocephalic shunts, must be made extremely soft and supple or else they will damage the body during long indwelling times resident in internal body compartments. Such devices, which are too soft and supple to respread such a predilated opening, must be inserted through the dilator or some other device which is stiffer. Such a device may be the one which has created the opening or it may be a second device which can hold open such a predilated opening created in the tissue. However, the maximum diameter of the device to be placed can be no larger than the minimum diameter of the interior passage inside the solid dilator or other hold-open device because of the need to ultimately slide the dilator or hold-open device off over the device that is being placed.

This latter requirement poses a problem for many devices. For example, both catheters and pacemakers leads are devices which terminate (at the end outside the body) in connectors which, for effective use, need to be much larger than the device (catheter or lead) itself. Pacemaker leads may actually be pre-attached to the pacemaker. To place such a device with a solid dilator, the dilator must be made with an internal passageway as large as the largest part of the device, in this case, the terminating connector. This creates problems. The hole through the tissue must be much larger than that required to accommodate passage of a catheter tube or a pacemaker, creating tissue trauma and disruption. The required hole may be so big that it cannot be created by spreading or pushing aside the tissue, but can only be cut surgically with a scalpel, thus requiring a return to the technique less preferred. When the thin part of the device is advanced through the dilator, there will now be much empty space between the device and the walls of the internal passageway of the dilator, creating a pathway for unintended and undesirable fluids to pass between compartments during the placement procedure. One example of this could be an air embolism entering a blood vessel between a small catheter and an oversized dilator during a catheter placement procedure, a potentially life-threatening complication.

A device which solves this problem is a peel away sheath, often used in conjunction with a solid dilator. The peel away sheath is a very thin-walled, ususally cylindrical device that is placed in position so that it provides a communicating passageway through the tissue. This is often accomplished by fitting the sheath tightly over a solid dilator, advancing both devices through the tissue together as a unit, then removing the solid dilator from inside the sheath, leaving the sheath alone in the desired position acting to hold the penetrated site in an open condition. Then the catheter tube or other instrument is advanced through the sheath into the desired position. The peel away sheath is capable of being readily and reliably pulled apart lengthwise into two pieces. This allows it to be made as small as the intercompartmental portion of the device, then removed by being pulled apart despite the presence of a (usually enlarged) connector or other end fitting. Proximal handles on the sheath are generally provided to facilitate grasping and tearing.

The types of peel away sheaths are known in the prior art. U.S. Patent Re 31,855, discloses a sheath, preferably of polytetrafluoroethylene material, that has an internal molecular orientation which tears easily in a lengthwise direction and with great difficulty in crosswise or oblique directions. This prevents an incomplete lengthwise tear which would result in an inability to remove the sheath. This sheath is furnished to the user already provided with pre-started, diametrically opposed longitudinal proximal tears. This sheath has the advantage of producing reliable tears. The tearing motion itself is easily performed by hand without the aid of a mechanical tearing device, is easily controlled, and has a good, smooth "feel" in use. Disadvantages include the severe limitation imposed on the materials that may be used for construction, which can only be materials capable of being fabricated with an oriented molecular structure. This makes it difficult to reliably attach handles, difficult to consistently fabricate a well-tapered tip to facilitate advancing the sheath through the tissue over the dilator, and difficult for the sheath to resist kinking when the sheath cylinder is bent while in use.

Patents Nos. 4,166,469, 4,243,050 and 4,345,606 disclose a sheath longitudinally scored on opposite sides. The result is a cylindrical sheath with two thinned areas (scored lines) which run lengthwise on opposite sides of the cylinder. It relies for its operation on the thinned or scored regions being mechanically weakened and, therefore, less resistant to tearing than the rest of the sheath cylinder, causing the tear, once started, to propogate along the weakened region and hence in a lengthwise fashion only. Advantages include a broader selection of materials permitting somewhat easier handle attachment, easier tip fabrication and better kink resistance. However, the same material is still used throughout, thus requiring that the material selected to fabricate the sheath possesses properties which are a compromise between the above-mentioned factors and longitudinal shear strength. Other disadvantages also include the difficulty of consistently fabricating a sufficiently weakened area to produce 100% consistent tears, less well-controlled and smooth tears and tears that have poorer "feel".

The present invention aims to overcome or substantially alleviate the aforestated problems and, in brief summary, provides a peel away hollow sheath for temporarily creating a passageway to a desired body site of a patient for placement of one end of an indwelling instrument at the body site through the sheath, the sheath comprising: a tube having a proximal end, a distal end and wall means defining a hollow interior, the wall means comprising at least two opposed axially-directed wall segments formed of material which has a relatively low resistance to shear and the remainder of the wall means being formed of material which has a relatively high resistance to shear. The sheath may also comprise opposed first and second grasping structures carried by the remainder of the wall means at the proximal end of the tube whereby, manually pulling the grasping structures apart tears the tube tears into two pieces axially along the low shear resistant segments.

In its preferred form the tube of the sheath is cylindrical, and may be formed in several way. It can be extruded as one continuous cylinder from two different extrusion materials using the process called simultaneous co-extrusion, then cut into lengths of suitable size for making peel away sheaths. Alternatively, semi-cylinders can be fabricated by extrusion or injection moulding and then joined via bridging T-shaped regions which have been injection-moulded using a process called insert moulding. Alternatively, the semi-cylinders and T square rods can be bonded together in sets using RF (radio frequency) welding or ultrasonic welding or gluing. Alternatively, the weakened regions can be formed by staggered co-extrusion in which the extrusion die first forms two semi-cylindrical regions, then forces them to join each other along two lines weakened because the extrusion material has already partially solidified and cannot form full, strong and effectively homogenous connecting regions, but instead forms the desired weakened region. Also, staggered extrusion may be used.

Additionally, the two materials may be the same basic plastic but with certain additives. The additive may be a plasticizer added to form the low shear resistant segments. Barium added to the material to be axially severed in proper proportion makes that material sufficiently weaker than the other material and at the same time provides the resulting device with radiopacity.

Conversely, certain additives, such as talc and calcium carbonate, in proper amounts may be added to the nontear material to make it adequately stronger than the tear material.

The tear material can be coloured different from the nontear material to aid in the manufacturing process, and to aid the user in identifying the sheath and in correctly using the sheath.

Embodiments of the invention thus provide a novel medical sheath comprising opposed axially-directed wall segments comprised of a material having a resistance to shear much lower than the remainder of the wall of the sheath for tearing the sheath into two pieces while still surrounding an indwelling instrument placed through the sheath.

In another aspect, the invention provides forming the low shear resistant segments independently of the remainder of the wall means and then joining the separate low shear resistant segments to the remainder of the wall means.

In order that the invention may be more readily understood, embodiments thereof will now be described, by way of example, with reference to the accompanying drawings, in which:

Figure 1 is a partly sectioned perspective view of a sheath embodying the present invention, being used to place a catheter tube in a vein of a patient;

Figure 2 is an enlarged cross-section taken along lines 2-2 of Figure 1;

Figure 3 is an enlarged cross-section taken along lines 3-3 of Figure 1; and

Figure 4 is an enlarged fragmentary transverse cross-section of a sheath having a tee-joint union between tear and nontear wall segments.

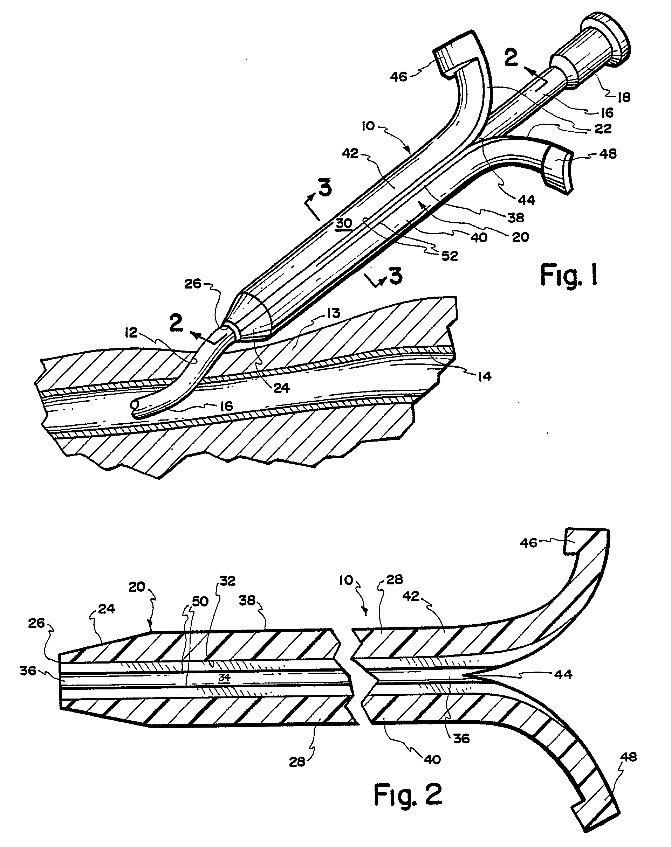

Reference is now made to the drawings, wherein like numerals are used to designate like parts throughout. Figures 1 to 3 illustrate a presently preferred medical peel away sheath, generally designated 10, fashioned in accordance with the principles of the present invention. Peel away sheath 10 comprises an integral bi-material device intended to temporarily preserve a tubular passageway from the exterior to a desired body site or internally between two body sites of a patient. Peel away sheath 10 is illustrated as having been retracted from a penetration site 12 from the exterior to a desired body site, i.e. through subcutaneous tissue 13 into a cardiovascular vein 14, where the sheath 10 earlier temporarily preserved a tubular passageway (the hollow interior of the sheath) at penetration site 12 while a pliant catheter tube 16 was inserted into the vein 14 through the hollow interior of the peel away sheath 10.

Retraction of the peel away sheath 10 to the position illustrated in Figure 1 is preliminary to severing the sheath 10 into two parts for removal of the same transversely from the catheter tube 16 thereby avoiding telescopic retraction over the proximal hub 18 at the trailing end of the catheter tube.

Typically, catheter tubes of the type used in conjunction with peel away sheaths are extremely soft and supple, having insufficient strength to be placed in a vein or the like using techniques which impose force upon the catheter tube. Such catheter tubes are often formed of silicone rubber or equivalent material, which is extremely compatible with human and animal tissue.

Notwithstanding the foregoing, it is to be appreciated that peel away sheaths of the type under consideration may be used not only in connection with access to a desired body site from the exterior of a patient, but in connection with access between two internal body sites.

The sheath 10 comprises a cylindrical tube 20 having a proximal end 22 and a distal end 24. Distal end 24 is illustrated as being in the form of a tapered tip having an axial port 26, the diameter of which is substantially the same as the outside diameter of the catheter tube 16. Other tip configurations, of course, can be used.

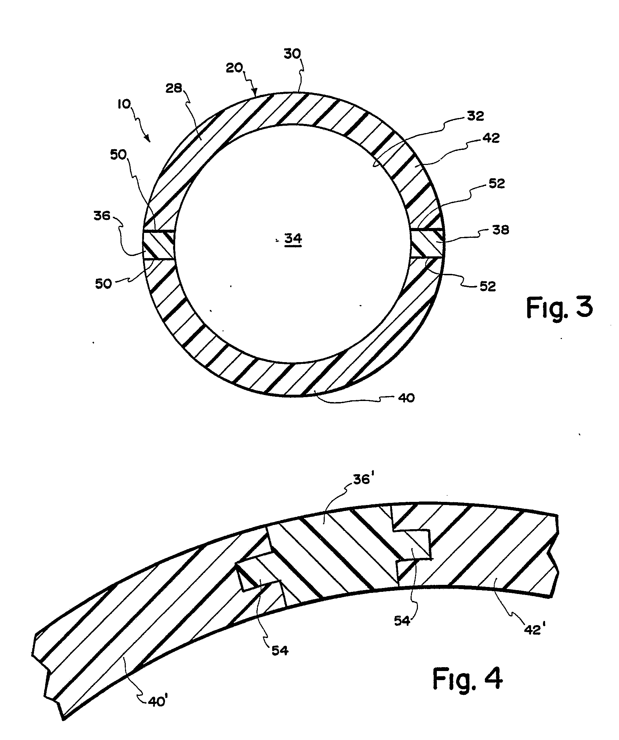

The tube 20 is comprised of synthetic resins. The tube 20 also comprises a wall 28 (Figure 3), which is illustrated as having a uniform thickness and comprising an exterior cylindrical surface 30 and an internal cylindrical surface 32. The resulting hollow interior 34 functions as a snug passageway for the catheter tube 16.

The wall 28 has two opposed axially-directed relative narrow wall segments or stripes 36 and 38, each formed of a material which has a relatively low reistance to shear. The remainder of the wall comprises two semi-cylindrical pieces 40 and 42, each of which is formed of a material having a relatively high resistance to shear.

Each segment 36, 38 comprises a V-shaped tear 44 at the proximal end of the sheath 10. The opposed V-shaped tears 44 are interposed between opposed first and second grasping structure or handles 46 and 48. Notches 44 serve to aid in starting the peeling or tearing process as hereinafter more fully described. Handles 46 and 48 are illustrated as being merely transverse flange-like enlargements of the semi-cylindrical wall portions 40 and 42. Once the peel away sheath 10 is in the position illustrated in Figure 1, the user merely grasps the handles or grasping structure 46 and 48 and pulls outwardly and distally causing the wall 20 to propagate the notches 44 as opposed tears axially-directed along the two low shear resistant segments 36 and 38 into two pieces thereby transversely removing the sheath 10 from a position circumscribing the indwelling catheter tube 16.

The low shear resistant segments 36 and 38 are illustrated in Figure 3 as joining with the semi-cylindrical segments 40 and 42, respectively, along axially extended, substantially radial interface lines 50 and 52. While, depending on the mode of fabrication, peel away sheath 10 can comprise substantially linear interfaces 50 and 52, the present invention is not restricted to such. For example, using certain modes of fabrication, the juncture sites between the low shear resistant segments and the semi-cylinders 40 and 42 can comprise T-shaped joints or tongue and groove connectors, as illustrated in Figure 4. Figure 4 is illustrated as comprising a low shear resistant segment 36′, similar to segment 36 heretofore described. Segment 36′ is interposed between two semi-cylindrical relatively high shear resistant semi-cylinders 40′ and 42′, each similar to cylinders 40 and 42. Tongue and groove or T-shaped connectors 54 join the axial interfaces of the segment 36′ to the axially directed edges of the semicylindrical portions 40′ and 42′. Other interface configuration can also be used without departing from the scope of the present invention.

It is presently preferred that the peel away sheath 10 be extruded as one continuous cylinder from two different extrusion materials using the process called simultaneous co-extrusion, following which the cylindrical tubing is cut into lengths and the tips 24, the notches 44 and handles 46 and 48 formed thereafter. For example, the semicylinders 40 and 42 may comprise polyethylene or Teflon and the segments 36 and 38 highly plasticized polyurethane. High density polypropylene or polyurethane may comprise semicylinders 40 and 42 and low density polypropylene or polyurethane may comprise the tear segments 36 and 38, respectively.

Alternatively, semicylinders 40 and 42 can be fabricated by extrusion or injection moulding and later joined via bridging T regions formed by injection moulding. The process of fusing the T regions to the semicylinders is called insert moulding.

Furthermore, the semicylinders 40 and 42 and low shear resistant T square rods can be bonded together in sets using RF or ultrasonic welding or a suitable adhesive or bonding agent.

The weakened regions may be formed by staggered co-extrusion in which the extrusion dies first form two semicylindrical regions then force the two regions to join each other along two lines weakened because the extrusion material is allowed to partially solidify and is weakened because it cannot form its full strength and effect homogeneous connection. Staggered extrusion may also be used.

The two materials may be the same basic synthetic resinous material, but modified with certain additives. The additive, placed in the low shear resistant segments 36 and 38 can be a plasticizer. The relative strengths of various synthetic resinous materials are well known in the art as are the effect of additives on strength. Calcium carbonate or talc added to most applicable synthetic resinous material, including polypropylene, in an amount of about 30% or more up to about 75% by weight may be used to produce the weakened segments 36 and 38 and in an amount from 1% to about 20% by weight may be used to produce the strengthened semicylinders 40 and 42. Barium can be added only to the material forming the low shear segments, preferably in a range of 30-75 percent by weight, which makes the segments 36 and 38 sufficiently weaker than the same material without the barium used to form the semi-cylinders 40 and 42. At the same time, the barium provides the resulting device with radiopacity. Fiberglass and/or glass beads may be added to the high shear strength semicylinders 40 and 42.

Conventional technology may be used to colour code the tear segments 36 and 38 and/or the semicylinders 40 and 42.

The features disclosed in the foregoing description, in the claims and/or in the accompanying drawings may, both separately and in any combination thereof, be material for realising the invention in diverse forms thereof.

## Claims

1. A peel away hollow sheath (10) for temporarily creating a passageway to a desired body site of a patient for placement of one end of an indwelling device at the body site through the sheath, the sheath comprising: tube means (20) having a proximal end (22), a distal end (24) and wall means (28) defining a hollow interior (34), the wall means (28) comprising at least two opposed axially-directed wall segments (36, 38) formed of material which has a relatively low resistance to shear and the remainder (40, 41) of the wall means being formed of material which has a relatively high resistance to shear; and opposed first and second grasping means (46, 41) carried by the remainder (40, 42) of the wall means at the proximal end (22) of the tube means (20) whereby manually pulling the grasping means (46, 48) apart tears the tube means (20) into two pieces axially along the low shear resistant segments (36, 38).

2. A peel away sheath according to claim 1, wherein the low shear resistant segments (36, 38) are formed of a first synthetic resinous material and the high shear resistant remainder (40, 42) of the wall means is formed of a second synthetic resinous material.

3. A peel away sheath according to claim 1 or 2, wherein the low shear resistant segments (36, 38) are co-extruded with the high shear resistant remainder (40, 42) of the wall means.

4. A peel away sheath according to claim 1 or 2, wherein the low shear resistant segments (36, 38) are axially joined to the high shear resistant remainder (40, 42) of the wall means by tee-joint connecting means.

5. A peel away sheath according to claim 1, wherein the low shear resistant segments (36, 38) and the high shear resistant remainder (40, 42) of the wall means are made from the same synthetic resinous material with a shear strength increasing additive added to the material from which the high shear resistant remainder (40, 42) of the wall means is formed and/or a shear strength reducing additive added to the material from which the low shear resistant segments (36, 38) are formed.

6. A peel away sheath according to claim 5, wherein the material forming the high shear resistant remainder (40, 42) of the wall means contains calcium carbonate or talc in the range of 1-20% by weight.

7. A peel away sheath according to claim 5 or 6, wherein the material forming the low shear resistant segments contains calcium carbonate or talc in the range of 30-75% by weight.

8. A peel away sheath according to claim 5, wherein the material from which the low shear resistant segments (36, 38) are formed contains plasticizer means.

9. A peel away sheath according to claim 5, wherein the material from which the low shear resistant segments (36, 38) are formed contains radiopaquing means.

10. A peel away sheath according to claim 5, wherein the material from which the low shear resistant segments (36, 38) are formed contains barium.

11. A peel away sheath according to any preceding claim, wherein the wall means are selectively colour coded.

12. A method of making a peel away hollow sheath (10) used to temporarily create a passageway into a desired body site of a patient for placement of one end of an indwelling device at the body site through the sheath, comprising the steps of: forming at least two opposed axially-directed wall segments (36, 38) of wall means (20) of the hollow sheath from material which has a relatively low resistance to shear and forming the remainder (40, 42) of the wall means from material having a relatively high resistance to shear, whereby oppositely pulling the remainder of the wall means tears the tube means into two pieces axially along the low shear resistant segments.

13. A method according to claim 12, comprising forming low shear resistant segments (36, 38) and the high shear resistant remainder of the wall means by simultaneous or staggered co-extrusion.

14. A method according to claim 12, comprising forming the low shear resistant segments (36, 38) independently of the remainder (40, 42) of the wall means and then joining the separate low shear resistant segments to the remainder of the wall means.

15. A method according to claim 12, 13 or 14, comprising using a first synthetic resinous material to form the low shear resistant segments (36, 38) and using a second synthetic resinous material to form the high shear resistant remainder (40, 42) of the wall means.

16. A method according to claim 12, 13 or 14, comprising using the same synthetic resinous material to form the low shear resistant segments (36, 38) and the high shear resistant remainder (40, 42) of the wall means, and adding a shear strength increasing additive to the material from which the high shear resistant remainder of the wall means is formed and/or a shear strength reducing additive to the material from which the low shear resistant segments are formed.

17. A method according to claim 16, comprising adding talc or calcium carbonate within the range of 1-20% by weight to the material from which the high shear resistant remainder (40, 42) of the wall means is formed.

18. A method according to claim 16 or 17 comprising adding talc or calcium carbonate within the range of 30-75% by weight to the material from which the low shear resistant segments (36, 38) are formed.

19. A method according to claim 16, comprising adding a plasticizer material to the material from which the low shear resistant segments (36, 38) are formed.

20. A method according to claim 16, comprising adding a radiopaquing means to the material from which the low shear resistant segments (36, 38) are formed.

Fig. 1

Fig. 2

Fig. 3

Fig. 4